# EUROPÄISCHE PATENTSCHRIFT

(11) **EP 0 475 254 B1**
(45) Veröffentlichungstag und Bekanntmachung des Hinweises auf die Patenterteilung: **28.12.1994**
(21) Anmeldenummer: 91114869.0
(22) Anmeldetag: 03.09.1991
(51) Int. Cl.: C07C 43/174, C09K 19/30

(54) **Chlorphenylalkoxyalkyl-Verbindungen**
Chlorophenyl-alkoxyalkyl-compounds
Composés de chlorophényl alkoxyalkyl

(30) Priorität: 10.09.1990 CH 2931/90
(43) Veröffentlichungstag der Anmeldung: 18.03.1992
(73) Patentinhaber: F. HOFFMANN-LA ROCHE AG, 4002 Basel (CH); DAINIPPON INK AND CHEMICALS, INC., Itabashi-ku Tokyo (JP)
(72) Erfinder: Buchecker, Richard, CH-8008 Zürich (CH); Schadt, Martin, CH-4411 Seltisberg (CH); Takatsu, Haruyoshi, Kodaira-shi, Tokyo (JP)
(74) Vertreter: Lederer, Franz, Dr.

(56) Entgegenhaltungen:
- EP-A- 0 415 090
- WO-A-90/15114
- DE-A- 4 025 418
- DE-A- 4 027 840

## Beschreibung

Die vorliegende Erfindung betrifft neue Chlorphenylalkoxyalkyl-Verbindungen, deren Herstellung, flüssigkristalline Gemische, die diese Verbindungen enthalten, sowie die Verwendung für elektro-optische Zwecke.

Flüssige Kristalle werden vor allem als Dielektrika in Anzeigevorrichtungen eingesetzt, da die optischen Eigenschaften dieser Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference") und neuerdings im Hinblick auf das Interesse an aktiv adressierten Flüssigkristallanwendungen auch TFT-Zellen (thin film transistor). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine hohe Stabilität gegenüber elektrischen Feldern und elektromagnetischer Strahlung besitzen. Sie sollten niedere Viskosität aufweisen und in den Zellen kurze Ansprechzeiten, tiefe Schwellenspannungen und einen hohen Kontrast ergeben. Weiterhin sollten sie bei üblichen Betriebstemperaturen eine geeignete Mesophase besitzen, beispielsweise eine nematische, cholesterische oder chiral getiltete smektische Phase. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Flüssigkristallmaterialien mit hoher optischer Anisotropie besteht in letzter Zeit ein vermehrtes Interesse an Materialien mit niedriger optischer Anisotropie, insbesondere für aktiv adressierte Flüssigkristallanzeigen, z.B. bei TFT-Anwendungen (Dünnfilmtransistor) in Fernsehgeräten.

Flüssigkristalle werden zwecks Optimierung der Eigenschaften in der Regel als Mischungen mehrerer Komponenten verwendet. Es ist daher wichtig, dass die Komponenten untereinander gut mischbar sind.

Gegenstand der vorliegenden Erfindung sind Verbindungen der allgemeinen Formel 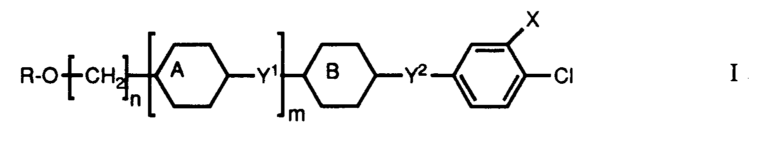
worin R einen Alkylrest mit 1 bis 12 Kohlenstoffatomen bezeichnet; n eine ganze Zahl von 2 bis 7 ist, Ring A trans-1,4-Cyclohexylen oder 1,4-Phenylen bezeichnet; m entweder 0 oder 1 ist; Ring B trans-1,4-Cyclohexylen, 1,4-Phenylen in welchem gegebenenfalls eine oder zwei nicht benachbarte CH-Gruppen durch Stickstoff ersetzt sind, oder, wenn m für 1 steht auch trans-1,3-Dioxan-2,5-diyl darstellt; Y¹ und Y² unabhängig voneinander eine einfache Kovalenzbindung oder -CH₂CH₂- darstellen; und X Wasserstoff, Fluor oder Chlor bezeichnet.

Die erfindungsgemässen Verbindungen sind Flüssigkristalle mit vergleichsweise hohen Klärpunkten und breiter nematischer Phase. Es wurde überraschenderweise gefunden, dass die Verbindungen der Formel I trotz der voluminösen Chlor-Endgruppe vergleichsweise kurze Schaltzeiten, insbesondere in Anzeigevorrichtungen mit nematischer Struktur, aufweisen.

Die erfindungsgemässen Verbindungen der Formel I eignen sich dank ihrer hohen spezifischen Widerstände, ihrer geringen Ionenlöslichkeit und den obenerwähnten vergleichsweise kürzeren Schaltzeiten speziell für TFT-Anwendungen.

Der Ausdruck "1,4-Phenylen, in welchem gegebenenfalls eine oder zwei nicht benachbarte CH-Gruppen durch Stickstoff ersetzt sind", umfasst in der vorliegenden Erfindung z.B. Gruppen wie 1,4-Phenylen, Pyridin-2,5-diyl oder Pyrimidin-2,5-diyl.

Vorzugsweise bezeichnet Ring B trans-1,4-Cyclohexylen oder 1,4-Phenylen.

Ganz besonders bevorzugt sind Verbindungen der Formel I worin Ring A 1,4-Cyclohexylen bezeichnet.

Der Ausdruck "Alkyl" umfasst geradkettige oder verzweigte Alkylreste mit 1 bis 12 Kohlenstoffatomen.

Ein bevorzugter Rest R ist ein geradkettiger Alkylrest mit 1 bis 5 Kohlenstoffatomen, besonders bevorzugt wird ein geradkettiger Alkylrest mit 1 bis 3 Kohlenstoffatomen.

Bevorzugte Verbindungen der Formel I sind Verbindungen, in welchen n eine ganze Zahl von 2 bis 5 ist. Weiter werden Verbindungen bevorzugt, worin mindestens eine der Gruppen Y¹ und Y² eine einfache Kovalenzbindung bedeutet.

Bevorzugte Verbindungen der Formel I sind die Verbindungen der allgemeinen Formeln 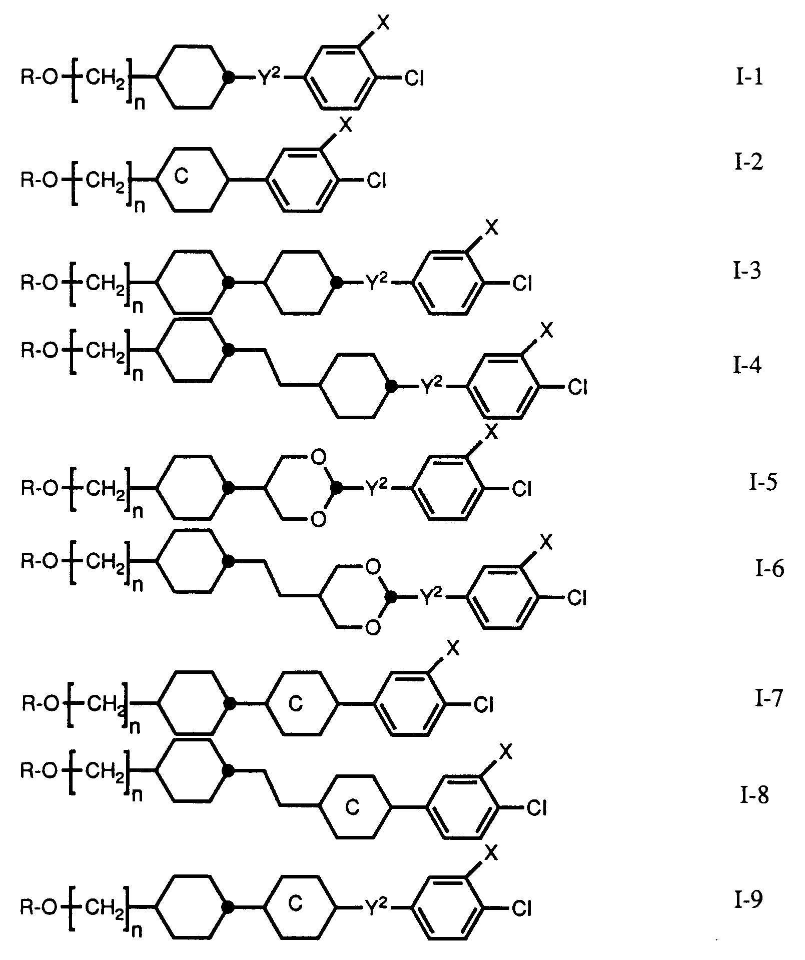
worin R, Y² und X die obengenannten Bedeutungen haben; und Ring C 1,4-Phenylen, Pyridin-2,5-diyl oder Pyrimidin-2,5-diyl darstellt.

Vorzugsweise steht in den Formeln I-2, I-7 und I-8 der Ring C für 1,4-Phenylen.

Die Herstellung der Verbindungen der Formel I kann in an sich bekannter Weise erfolgen. Bevorzugte Methoden werden anhand der folgenden Schemata veranschaulicht, worin R und B die obengenannte Bedeutung haben und n für eine ganze Zahl von 1 bis 3 steht.

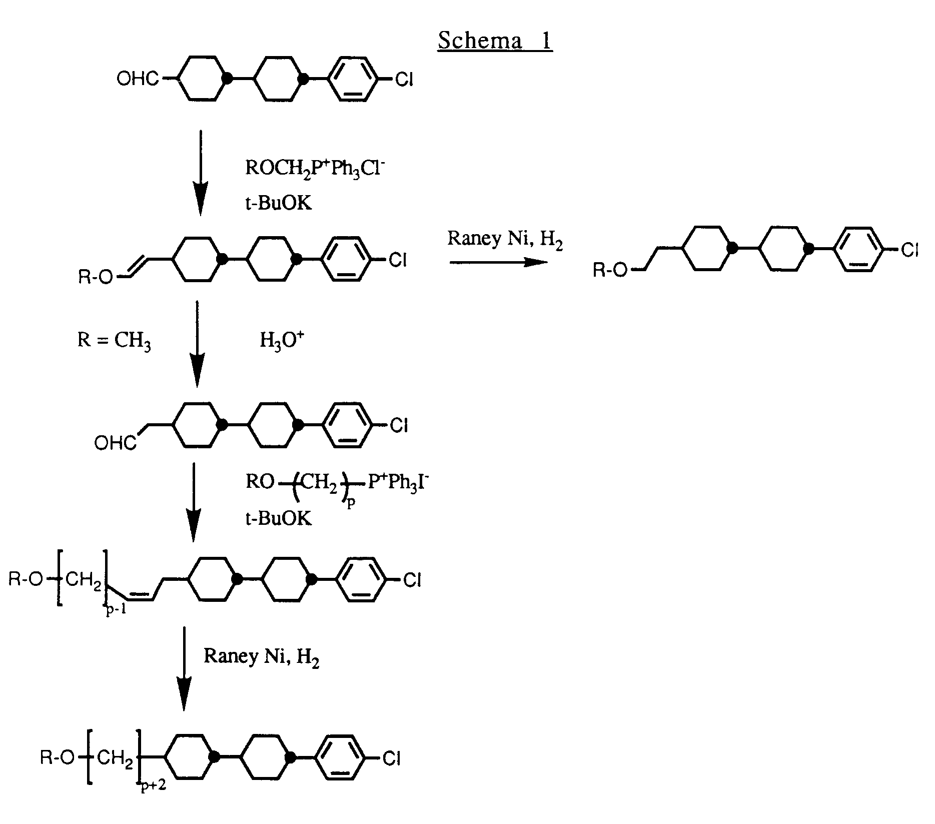

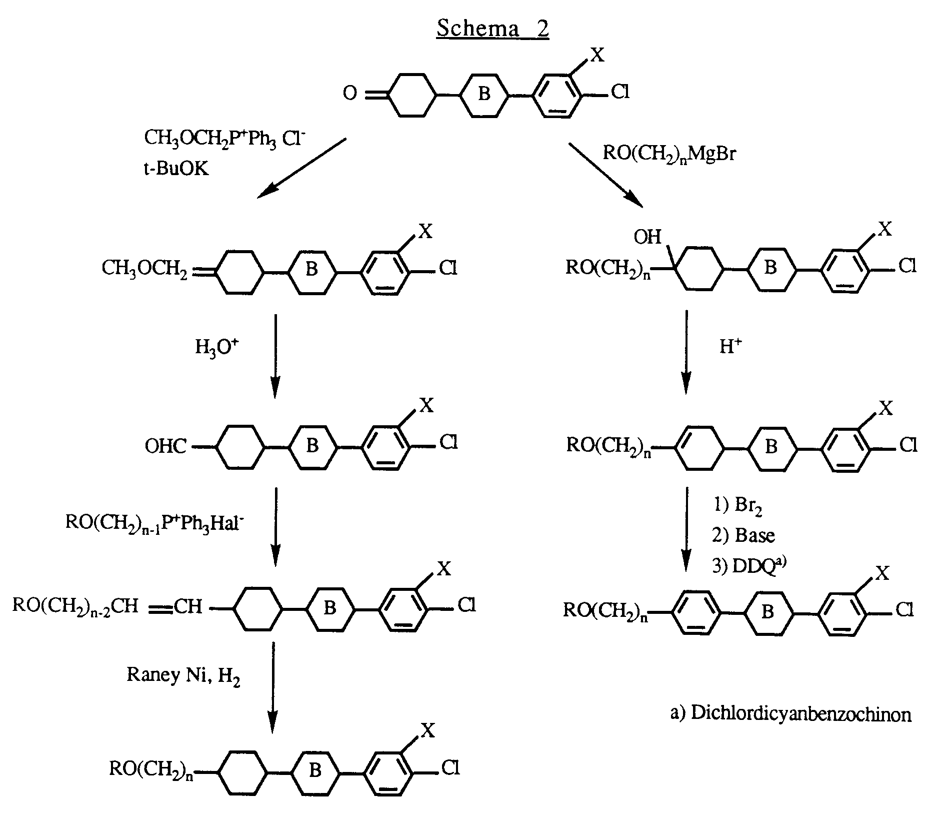

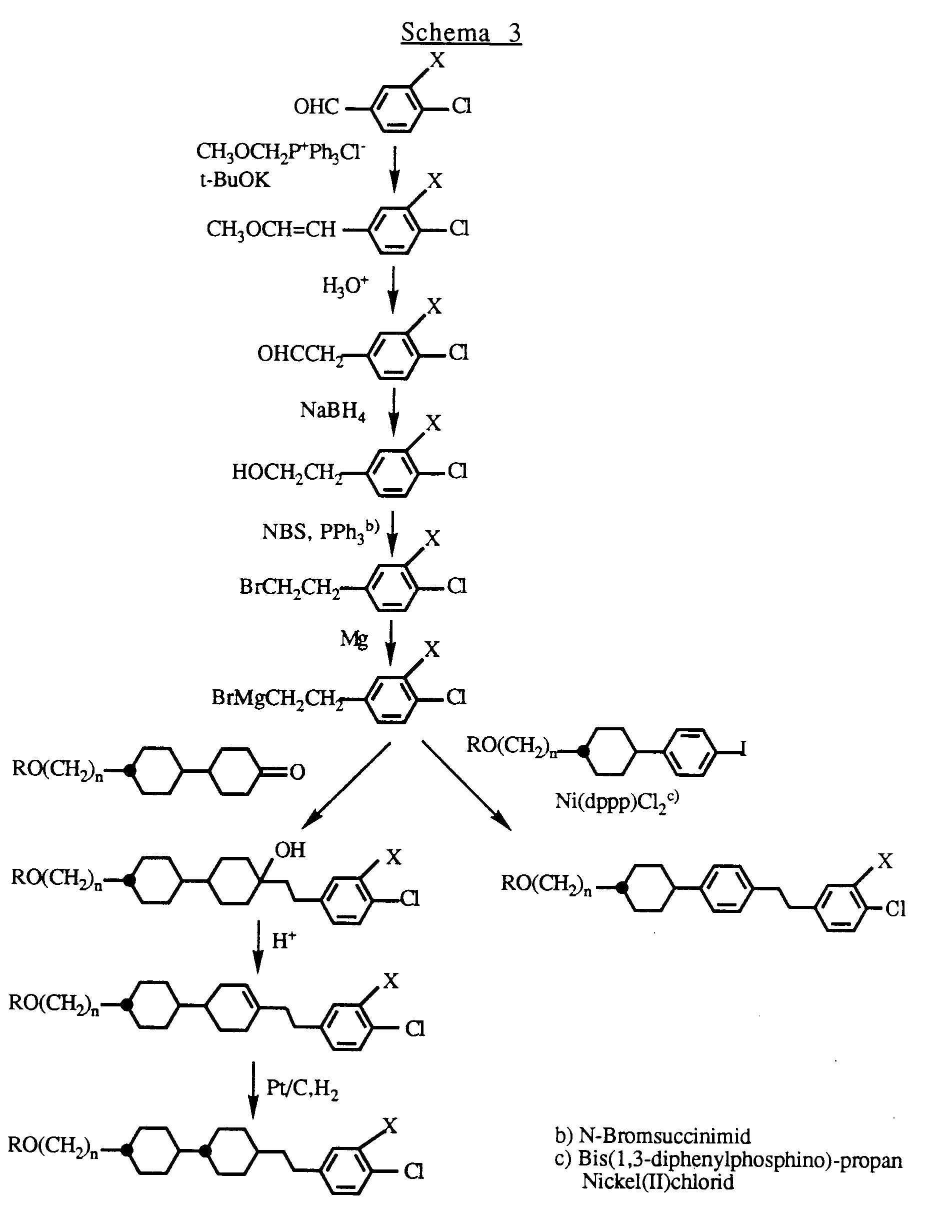

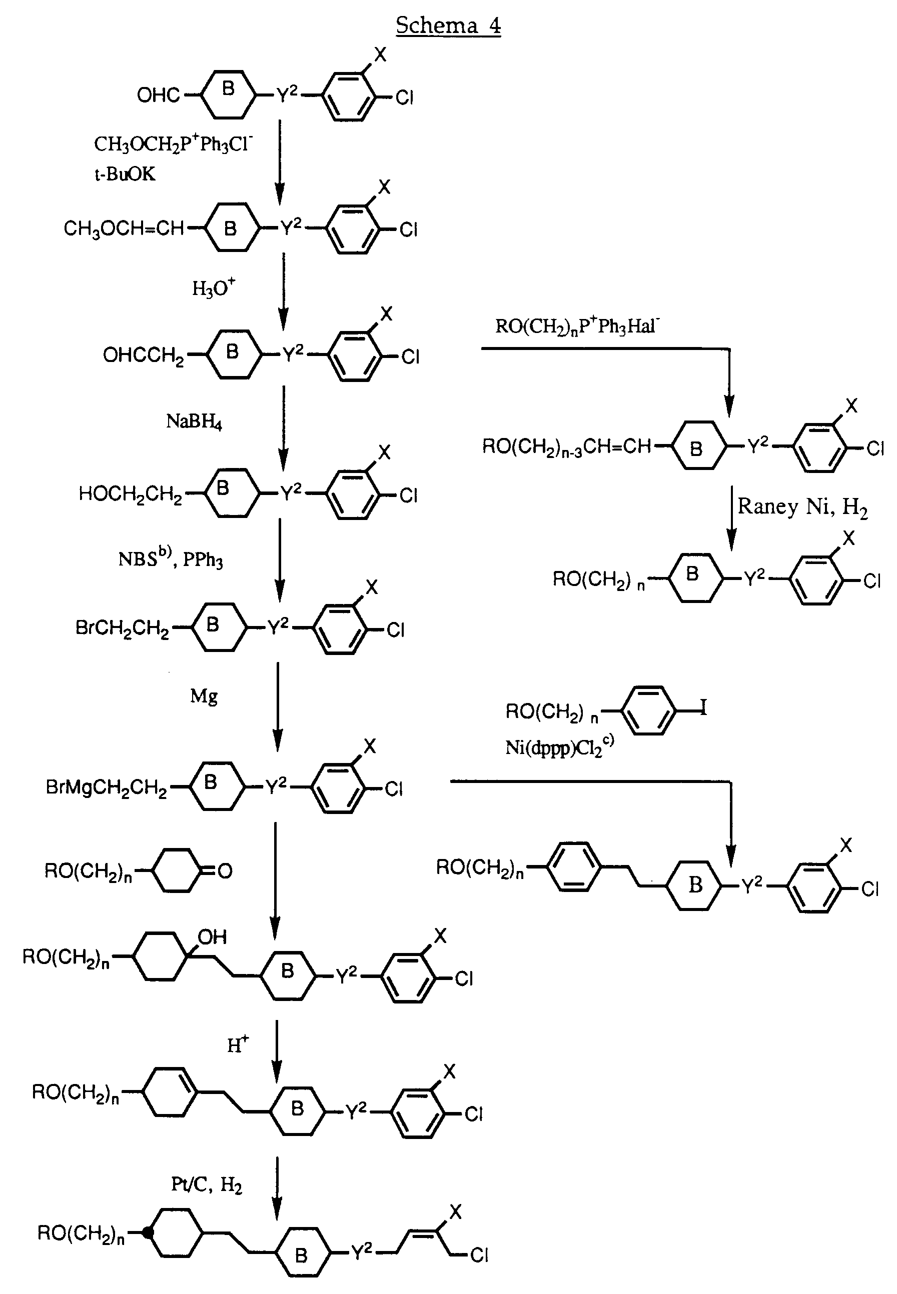

R, X, n, Ring B, Y² haben in den Schemata 1-4 die oben angegebene Bedeutung; p ist eine ganze Zahl von 1 bis 5.

Die Ausgangsmaterialien sind bekannte oder Analoge bekannter Verbindungen und können nach bekannten Methoden hergestellt werden.

Die erfindungsgemässen Verbindungen der Formel I sind gut untereinander und/oder mit anderen Flüssigkristall-Komponenten mischbar. Sie führen in flüssigkristallinen Gemischen zu einer breiten nematischen Mesophase, niedriger Schwellenspannung und kurzen Ansprechzeiten.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I oder andere Flüssigkristallkomponenten sein. Aufgrund der guten Löslichkeit der Verbindungen der Formel I und aufgrund ihrer guten Mischbarkeit untereinander kann ihr Anteil in den erfindungsgemässen Gemischen relativ hoch sein. Im allgemeinen ist jedoch ein Anteil von etwa 1-50 Gew.-%, insbesondere etwa 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formel 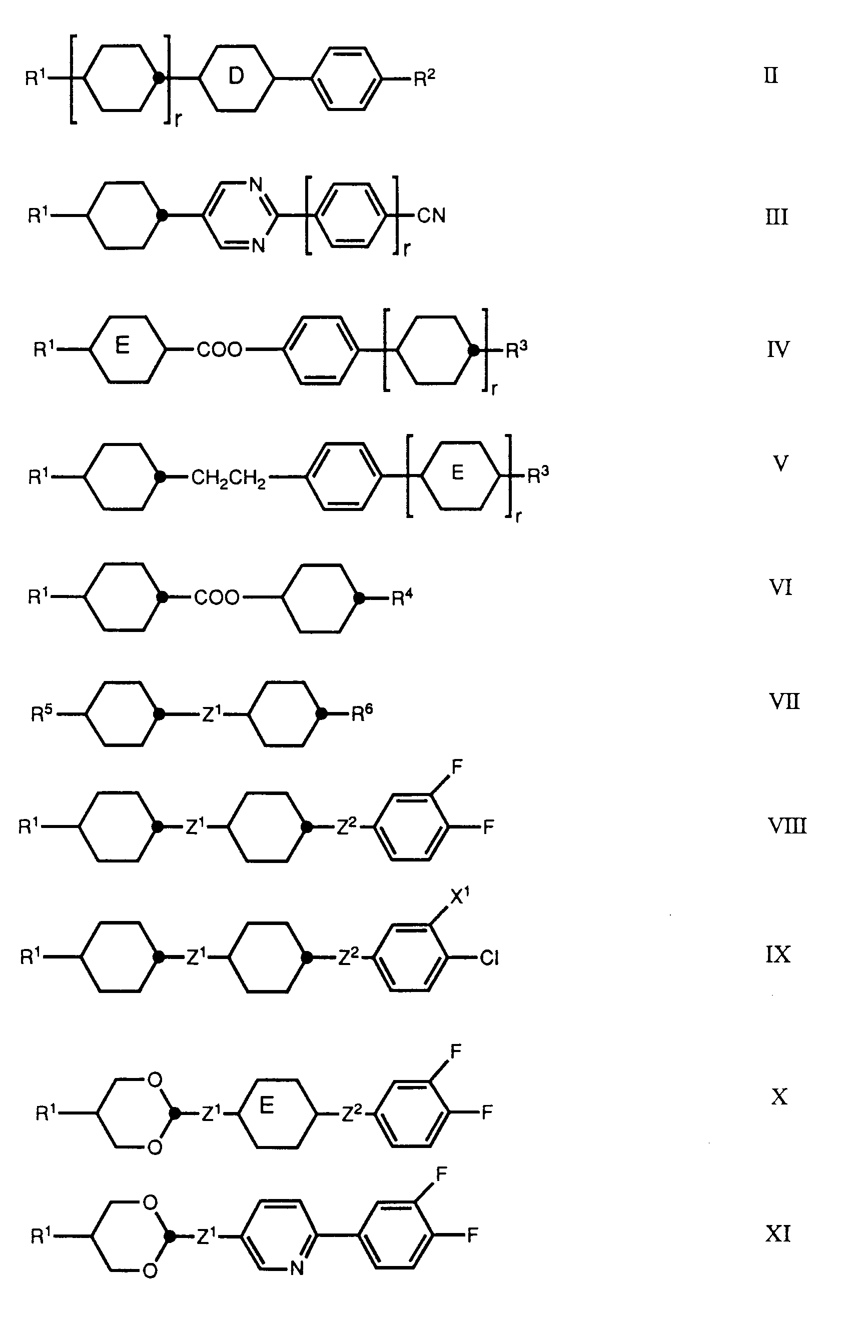 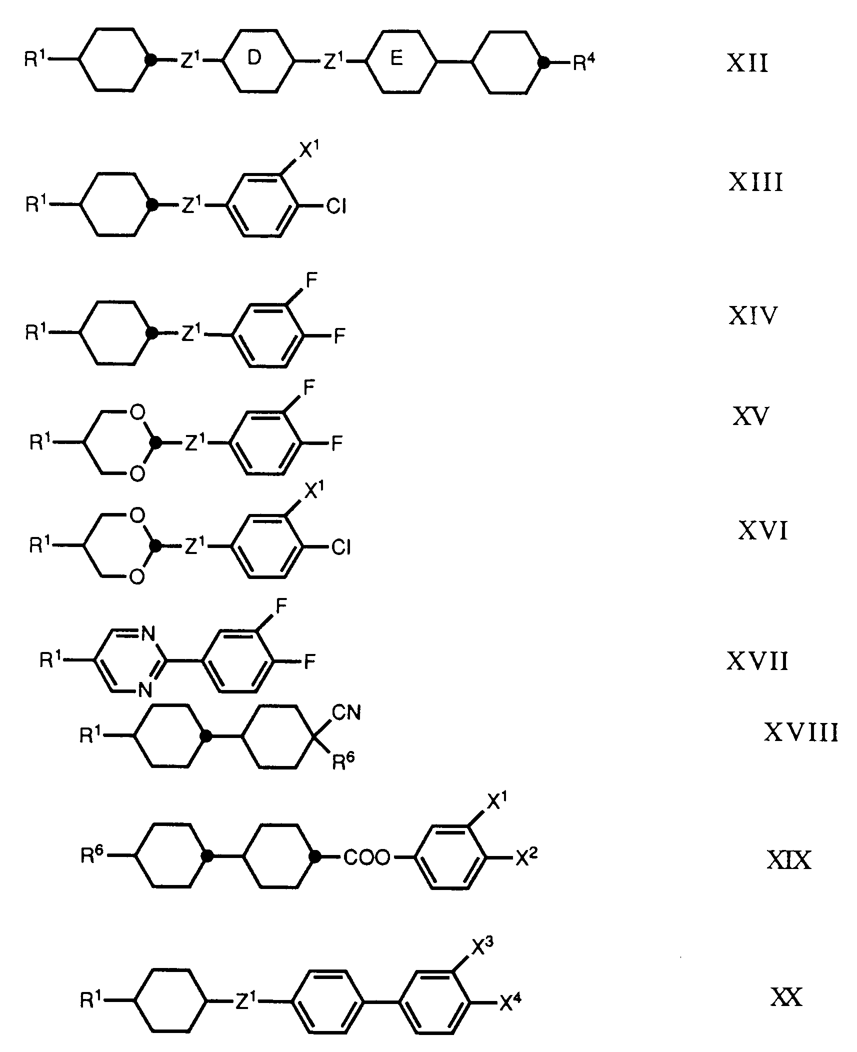
worin r für die Zahl 0 oder 1 steht; R¹ und R⁴ unabhängig voneinander Alkyl, Alkoxyalkyl, 3E-Alkenyl, 4-Alkenyl oder an einem gesättigten Ring auch 1E-Alkenyl bedeutet; Ring D 1,4-Phenylen, trans-1,4-Cyclohexylen, Pyridin-2,5-diyl, Pyrimidin2,5-diyl oder trans-1,3-Dioxan-2,5-diyl darstellt; R² Cyano, -NCS, Fluor, Alkyl, 3E-Alkenyl, 4-Alkenyl, Alkoxy, 2E-Alkenyloxy, 3-Alkenyloxy oder 1-Alkinyl bezeichnet; Ring E 1,4-Phenylen oder trans-1,4-Cyclohexylen darstellt;
R³ Alkyl, 3E-Alkenyl, 4-Alkenyl oder an einem Cyclohexanring auch 1E-Alkenyl oder an einem Benzolring auch Cyano, -NCS, Alkoxy, 2E-Alkenyloxy oder 3-Alkenyloxy bedeutet; R⁵ Alkyl, 1E-Alkenyl, 3E-Alkenyl oder 4-Alkenyl bezeichnet; Z¹ und Z² unabhängig voneinander eine einfache Kovalenzbindung oder -CH₂CH₂- darstellen, wobei zwei aromatische Ringe immer durch eine einfache Kovalenzbindung getrennt sind; R⁶ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet; X¹ Wasserstoff, Chlor oder Fluor bezeichnet; X² Cyano, Chlor oder Fluor bedeutet; X³ Wasserstoff oder Fluor bedeutet; und X⁴ Chlor oder Fluor bezeichnet.

Der obige Ausdruck "gesättigter Ring" umfasst trans-1,4-Cyclohexylen und trans-1,3-Dioxan-2,5-diyl. Reste R¹ bis R⁶ besitzen vorzugsweise höchstens je 12 Kohlenstoffatome, besonders bevorzugt höchstens je 7 Kohlenstoffatome. Geradkettige Reste sind im allgemeinen bevorzugt.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. In den Beispielen bedeutet C eine kristalline, N eine nematische und I die isotrope Phase. V₁₀ bezeichnet die Spannung für 10% Transmission. Tₒₙ und T_{off} bezeichnen die Einschaltzeit bzw. die Ausschaltzeit. Δn bezeichnet die optische Anisotropie.

### Beispiel 1

In einem Autoklaven werden 5,4 g 1-Chlor-4-[trans-4-(5-methoxy-2-pentenyl)cyclohexyl]benzol in 80 ml Aethylacetat gelöst, mit 1 g Raney-Nickel versetzt und während 7 Stunden bei 60°C, 5 kg/cm² hydriert. Die Suspension wird filtriert und eingedampft. Der Rückstand (5,2 g) wird zweimal aus je 30 ml Aethanol kristallisiert und ergibt 3,4 g 1-Chlor-4-[trans-4-(5-methoxypentyl)cyclohexyl]benzol; Smp. (C-I) 52,8°C.

Das als Ausgangsmaterial verwendete 1-Chlor-4-[trans4-(5-methoxy-2-pentenyl)cyclohexyl]benzol wird auf folgende Weise hergestellt:
a) Eine mit Stickstoff begaste Suspension von 9,3 g (Methoxymethyl)triphenylphosphoniumchlorid in 40 ml Tetrahydrofuran wird auf -5°C gekühlt und mit 3,9 g Kalium-tert.-Butylat versetzt. Die Suspension wird 10 Minuten bei -5°C, 1 Stunde bei Raumtemperatur gerührt, wieder auf -5°C gekühlt und innert 5 Minuten mit einer Lösung von 5 g trans-4-(4-Chlorphenyl)-cyclohexancarboxaldehyd in 20 ml Tetrahydrofuran versetzt. Nach 10 Minuten Rühren bei -5°C und 1 Stunde bei Raumtemperatur werden 50 ml Wasser hinzugefügt und die wässrige Phase mit 100 ml Aethylacetat extrahiert. Die organische Phase wird mit Wasser gewaschen, über Natriumsulfat getrocknet, filtriert und eingedampft. Der Rückstand wird mit 100 ml Hexan 1 Stunde gerührt, filtriert und mit 50 ml Hexan gewaschen. Das Filtrat wird eingeengt und ergibt 5 g 1-Chlor-4-[trans-4-(2-methoxyvinyl)cyclohexyl]benzol.
b) 5 g 1-Chlor-4-[trans-4-(2-methoxyvinyl)cyclohexyl]benzol werden in 60 ml Tetrahydrofuran und 20 ml 9-proz. Salzsäure während 2 Stunden bei 60°C gerührt. Nach Abkühlen auf Raumtemperatur wird die wässrige Phase mit 100 ml Aethylacetat extrahiert. Die organische Phase wird mit Wasser gewaschen, über Natriumsulfat getrocknet, filtriert und eingedampft. Der Rückstand ergab 4,6 g [trans-4-(4-Chlorphenyl)cyclohexyl]acetaldehyd.
c) Eine mit Stickstoff begaste Suspension von 11,4 g (3-Methoxypropyl)triphenylphosphoniumjodid in 50 ml Tetrahydrofuran wird auf -5°C gekühlt, mit 3,36 g Kalium-tert.-butylat versetzt, 10 Minuten bei -5°C und 1 Stunde bei Raumtemperatur gerührt. Nach Abkühlen des Reaktionsgemisches auf -5°C wird innert 5 Minuten eine Lösung von 4,6 g [trans-4-(4-Chlorphenyl)-cyclohexyl]acetaldehyd in 25 ml Tetrahydrofuran zugetropft, 10 Minuten bei -5°C und 1 Stunde bei Raumtemperatur gerührt, mit 50 ml Wasser versetzt und die wässrige Phase mit 100 ml Aethylacetat extrahiert. Die organische Phase wird mit Wasser gewaschen, über Natriumsulfat getrocknet, filtriert und eingeengt. Der Rückstand wird in 50 ml Toluol aufgenommen, durch Erwärmen auf 70°C und Rühren während 30 Minuten gelöst. Die Lösung wird auf Raumtemperatur gekühlt, mit 50 ml Hexan versetzt, 2 Stunden gerührt, filtriert und mit 30 ml Toluol/Hexan (1:1 Vol.) gewaschen. Das Filtrat wird über 100 g Kieselgel filtriert, das Kieselgel mit 200 ml Hexan/Toluol (1:1 Vol.) gewaschen. Das Filtrat wird eingeengt und ergibt 5,4 g 1-Chlor-4-[trans-4-(5-methoxy-2-pentenyl)cyclohexyl]benzol.

Auf analoge Weise können folgende Verbindungen hergestellt werden:
1-Chlor-4-[trans-4-(3-methoxypropyl)cyclohexyl]benzol,
1-Chlor-4-[trans-4-(3-äthoxypropyl)cyclohexyl]benzol,
1-Chlor-4-[trans-4-(3-propoxypropyl)cyclohexyl]benzol,
1-Chlor-4-[trans-4-(4-methoxybutyl)cyclohexyl]benzol,
1-Chlor-4-[trans-4-(4-äthoxybutyl)cyclohexyl]benzol,
1-Chlor-4-[trans-4-(5-äthoxypentyl)cyclohexyl]benzol,
1,2-Dichlor-4-[trans-4-(3-methoxypropyl)cyclohexyl]benzol,
1,2-Dichlor-4-[trans-4-(3-äthoxypropyl)cyclohexyl]benzol,
1,2-Dichlor-4-[trans-4-(3-propoxypropyl)cyclohexyl]benzol,
1,2-Dichlor-4-[trans-4-(4-methoxybutyl)cyclohexyl]benzol,
1,2-Dichlor-4-[trans-4-(4-äthoxybutyl)cyclohexyl]benzol,
1,2-Dichlor-4-[trans-4-(5-methoxypentyl)cyclohexyl]benzol,
1-Chlor-2-fluor-4-[trans-4-(3-methoxypropyl)cyclohexyl]benzol,
1-Chlor-2-fluor-4-[trans-4-(3-äthoxypropyl)cyclohexyl]benzol,
1-Chlor-2-fluor-4-[trans-4-(3-propoxypropyl)cyclohexyl]benzol,
1-Chlor-2-fluor-4-[trans-4-(4-methoxybutyl)cyclohexyl]benzol,
1-Chlor-2-fluor-4-[trans-4-(4-äthoxybutyl)cyclohexyl]benzol,
1-Chlor-2-fluor-4-[trans-4-(5-methoxypentyl)cyclohexyl]benzol,
2-(p-Chlorphenyl)-5-(3-methoxypropyl)pyridin,
2-(p-Chlorphenyl)-5-(5-methoxypentyl)pyridin,
2-(p-Chlorphenyl)-5-(3-methoxypropyl)pyrimidin,
2-(p-Chlorphenyl)-5-(5-methoxypentyl)pyrimidin,
4-Chlor-4'-(3-methoxypropyl)biphenyl,
4-Chlor-4'-(5-methoxypentyl)biphenyl,
4-Chlor-3-fluor-4'-(3-methoxypropyl)biphenyl.

### Beispiel 2

In einem Autoklaven wird ein Gemisch aus 4,6 g trans4-(4-Chlorphenyl)-trans-4'-(3-methoxy -2-propenyl)[1,1'-bicyclohexyl] in 80 ml Aethylacetat und 1 g Raney-Nickel während 10 Stunden bei 60°C und 5 kg/cm² hydriert. Die Suspension wird filtriert und eingedampft. Der Rückstand (4,5 g) wird zweimal aus je 30 ml Aethanol umkristallisiert und ergibt 3,5 g trans-4-(4-Chlorphenyl)-trans -4'-(3-methoxypropyl)[1,1'-bicyclohexyl]; Smp. (C-N) 73°C, Klp. (N-I) 185,7°C.

Das als Ausgangsmaterial verwendete trans-4-(4-Chlorphenyl)-trans-4'-(3-methoxy -2-propenyl)[1,1'-bicyclohexyl] wurde auf folgende Weise hergestellt:
a) Eine mit Stickstoff begaste Suspension von 9,3 g (Methoxymethyl)triphenylphosphoniumchlorid in 40 ml Tetrahydrofuran wird auf -5°C gekühlt und mit 3,9 g Kalium-tert.-Butylat versetzt. Die Suspension wird 10 Minuten bei -5°C, 1 Stunde bei Raumtemperatur gerührt, wieder auf -5°C gekühlt und innert 5 Minuten mit einer Lösung von 12 g trans4'-(4-Chlorphenyl)-trans -4-[1,1'-bicyclohexyl]carboxaldehyd in 60 ml Tetrahydrofuran versetzt. Nach 10 Minuten Rühren bei -5°C und 1 Stunde bei Raumtemperatur werden 100 ml Wasser hinzugefügt und die wässrige Phase mit 200 ml Aethylacetat extrahiert. Die organische Phase wird mit Wasser gewaschen, über Natriumsulfat getrocknet, filtriert und eingedampft. Der Rückstand wird mit 100 ml Toluol aufgenommen, durch Erwärmen auf 70°C und Rühren während 30 Minuten gelöst. Die Lösung wird auf Raumtemperatur gekühlt, mit 200 ml Hexan versetzt, 2 Stunden gerührt, filtriert und mit 70 ml Hexan/Toluol (1:1 Vol.) gewaschen. Das Filtrat wird über 200 g Kieselgel filtriert, die Säule mit 500 ml Hexan/ Toluol (1:1 Vol.) gespült. Das Filtrat wird eingeengt und ergibt 11 g trans-4-(4-Chlorphenyl)-trans-4'-(2-methoxyvinyl)[1,1'-bicyclohexyl].
b) 5,5 g trans-4-(4-Chlorphenyl)-trans-4'-(2-methoxyvinyl)[1,1'-bicyclohexyl] werden in 60 ml Tetrahydrofuran und 20 ml 9-proz. Salzsäure während 3 Stunden bei 60°C gerührt. Nach Abkühlen auf Raumtemperatur wird die wässrige Phase mit 150 ml Aethylacetat extrahiert. Die organische Phase wird mit Wasser gewaschen, über Natriumsulfat getrocknet, filtriert und eingedampft. Der Rückstand ergab 5 g [trans-4'-(4-Chlorphenyl)[1,1'-bicyclohexyl]-4-trans-yl]acetaldehyd.
c) Eine mit Stickstoff begaste Suspension von 10,3 g (Methoxymethyl)triphenylphosphoniumchlorid in 50 ml Tetrahydrofuran wird auf -5°C gekühlt, mit 4,5 g Kalium-tert.Butylat versetzt, 10 Minuten bei -5°C und 1 Stunde bei Raumtemperatur gerührt. Nach Abkühlen des Reaktionsgemisches auf -5°C wird innert 5 Minuten eine Lösung von 5 g [trans-4'-(4-Chlorphenyl)[1,1'-bicyclohexyl]-4-trans-yl]acetaldehyd in 30 ml Tetrahydrofuran zugetropft, 10 Minuten bei -5°C und 1 Stunde bei Raumtemperatur gerührt, mit 50 ml Wasser versetzt und die wässrige Phase mit 100 ml Aethylacetat extrahiert. Die organische Phase wird mit Wasser gewaschen, über Natriumsulfat getrocknet, filtriert und eingeengt. Der Rückstand wird in 70 ml Toluol aufgenommen, durch Erwärmen auf 70°C und Rühren während 30 Minuten gelöst. Die Lösung wird auf Raumtemperatur gekühlt, mit 70 ml Hexan versetzt, 2 Stunden gerührt, filtriert und mit 40 ml Toluol/Hexan (1:1 Vol.) gewaschen. Das Filtrat wird über 100 g Kieselgel filtriert, das Kieselgel mit 200 ml Hexan/Toluol (1:1 Vol.) gewaschen. Das Filtrat wird eingeengt und ergibt 4,6 g trans-4-(4-Chlorphenyl)-trans4'-(3-methoxy-2-propenyl)[1,1'-bicyclohexyl].

Auf analoge Weise können folgende Verbindungen hergestellt werden:
trans-4-(4-Chlorphenyl)-trans-4'-(3-äthoxypropyl)[1,1'-bicyclohexyl],
trans-4-(4-Chlorphenyl)-trans-4'-(3-propoxypropyl)[1,1'-bicyclohexyl],
trans-4-(4-Chlorphenyl)-trans-4'-(4-methoxybutyl)[1,1'-bicyclohexyl],
trans-4-(4-Chlorphenyl)-trans-4'-(4-äthoxybutyl)[1,1'-bicyclohexyl],
trans-4-(4-Chlorphenyl)-trans-4'-(5-methoxypentyl)[1,1'-bicyclohexyl],
trans-4-(3,4-Dichlorphenyl)-trans-4'-(3-methoxypropyl)-[1,1'-bicyclohexyl],
trans-4-(3,4-Dichlorphenyl)-trans-4'-(3-äthoxypropyl)-[1,1'-bicyclohexyl],
trans-4-(3,4-Dichlorphenyl)-trans-4'-(3-propoxypropyl)-[1,1'-bicyclohexyl],
trans-4-(3,4-Dichlorphenyl)-trans-4'-(4-methoxybutyl)-[1,1'-bicyclohexyl],
trans-4-(3,4-Dichlorphenyl)-trans-4'-(4-äthoxybutyl)-[1,1'-bicyclohexyl],
trans-4-(3,4-Dichlorphenyl)-trans-4'-(5-methoxypentyl)-[1,1'-bicyclohexyl],
trans-4-(4-Chlor-3-fluorphenyl)-trans-4'-(3-methoxypropyl)[1,1'-bicyclohexyl],
trans-4-(4-Chlor-3-fluorphenyl)-trans-4'-(3-äthoxypropyl)[1,1'-bicyclohexyl],
trans-4-(4-Chlor-3-fluorphenyl)-trans-4'-(3-propoxypropyl)[1,1'-bicyclohexyl],
trans-4-(4-Chlor-3-fluorphenyl)-trans-4'-(4-methoxybutyl)[1,1'-bicyclohexyl],
trans-4-(4-Chlor-3-fluorphenyl)-trans-4'-(4-äthoxybutyl)[1,1'-bicyclohexyl],
trans-4-(4-Chlor-3-fluorphenyl)-trans-4'-(5-methoxypentyl)[1,1'-bicyclohexyl],
2-(p-Chlorphenyl)-5-[trans-4-(3-methoxypropyl)cyclohexyl]pyridin,
2-(p-Chlorphenyl)-5-[trans-4-(3-methoxypropyl)cyclohexyl]pyrimidin,
trans-2-(p-Chlorphenyl)-5-[trans-4-(3-methoxypropyl)-cyclohexyl]-1,3-dioxan,
2-(p-Chlorphenyl)-5-[4-(3-methoxypropyl)phenyl]pyridin,
2-(p-Chlorphenyl)-5-[4-(3-methoxypropyl)phenyl]pyrimidin,
4-Chlor-4'-[trans-4-(3-methoxypropyl)cyclohexyl]biphenyl,
4-Chlor-4'-[trans-4-(3-äthoxypropyl)cyclohexyl]biphenyl,
4-Chlor-4'-[trans-4-(4-methoxybutyl)cyclohexyl]biphenyl,
4-Chlor-4'-[trans-4-(4-äthoxybutyl)cyclohexyl]biphenyl,
4-Chlor-4'-[trans-4-(5-methoxypentyl)cyclohexyl]biphenyl,
4-Chlor-3-fluor-4'-[trans-4-(3-methoxypropyl)cyclohexyl]-biphenyl,
4-Chlor-3-fluor-4'-[trans-4-(3-äthoxypropyl)cyclohexyl]-biphenyl,
4-Chlor-3-fluor-4'-[trans-4-(4-methoxybutyl)cyclohexyl]-biphenyl,
4-Chlor-3-fluor-4'-[trans-4-(5-methoxypentyl)cyclohexyl]-biphenyl,
3,4-Dichlor-4'-[trans-4-(3-methoxypropyl)cyclohexyl]-biphenyl,
3,4-Dichlor-4'-[trans-4-(4-methoxybutyl)cyclohexyl]-biphenyl,
3,4-Dichlor-4'-[trans-4-(5-methoxypentyl)cyclohexyl]-biphenyl.

### Beispiel 3

In einem Autoklaven werden 5,5 g trans-4-(4-Chlorphenyl)-trans -4'-(2-methoxyvinyl)[1,1'-bicyclohexyl] in 80 ml Aethylacetat gelöst, mit 1 g Raney-Nickel versetzt und während 7 Stunden bei 60°C, 5 kg/cm² hydriert. Die Suspension wird filtriert, eingedampft und ergibt 5,4 g rohes trans-4-(4-Chlorphenyl)-trans -4'-(2-methoxyäthyl)[1,1'bicyclohexyl]; Smp. (C-N) 61,2°C, Klp. (N-I) 146°C.

Das als Ausgangsmaterial verwendete trans-4-(4-Chlorphenyl)-trans-4'-(2-methoxyvinyl)[1,1'-bicyclohexyl] wird auf folgende Weise hergestellt:
20,6 g Methoxymethyltriphenylphosphoniumchlorid und 12 g [trans-4'-(4-Chlorphenyl)[1,1'-bicyclohexyl]trans -4-yl]carboxaldehyd werden wie in Beispiel 2a) beschrieben umgesetzt und ergeben 11 g trans-4-(4-Chlorphenyl)-trans-4'(2-methoxyvinyl)[1,1'-bicyclohexyl].

Auf analoge Weise können folgende Verbindungen hergestellt werden:
1-Chlor-4-[trans-4-(2-methoxyäthyl)cyclohexyl]benzol,
1-Chlor-4-[trans-4-(2-äthoxyäthyl)cyclohexyl]benzol,
1-Chlor-4-[trans-4-(2-propoxyäthyl)cyclohexyl]benzol,
1-Chlor-4-[trans-4-(2-butoxyäthyl)cyclohexyl]benzol,
1,2-Dichlor-4-[trans-4-(2-methoxyäthyl)cyclohexyl]benzol,
1,2-Dichlor-4-[trans-4-(2-äthoxyäthyl)cyclohexyl]benzol,
1,2-Dichlor-4-[trans-4-(2-propoxyäthyl)cyclohexyl]benzol,
1-Chlor-2-fluor-4-[trans-4-(2-methoxyäthyl)cyclohexyl]-benzol,
1-Chlor-2-fluor-4-[trans-4-(2-äthoxyäthyl)cyclohexyl]-benzol,
1-Chlor-2-fluor-4-[trans-4-(2-propoxyäthyl)cyclohexyl]-benzol,
4-Chlor-4'-(2-methoxyäthyl)biphenyl,
4-Chlor-4'-(2-äthoxyäthyl)biphenyl,
4-Chlor-3-fluor-4'-(2-methoxyäthyl)biphenyl,
2-(4-Chlorphenyl)-5-(2-methoxyäthyl)pyridin,
2-(4-Chlorphenyl)-5-(2-äthoxyäthyl)pyridin,
2-(4-Chlorphenyl)-5-(2-methoxyäthyl)pyrimidin,
2-(4-Chlorphenyl)-5-(2-äthoxyäthyl)pyrimidin,
trans-4-(4-Chlorphenyl)-trans-4'-(2-methoxyäthyl)[1,1'-bicyclohexyl],
trans-4-(4-Chlorphenyl)-trans-4'-(2-äthoxyäthyl)[1,1'-bicyclohexyl],
trans-4-(4-Chlorphenyl)-trans-4'-(2-propoxyäthyl)[1,1'-bicyclohexyl],
trans-4-(3,4-Dichlorphenyl)-trans-4'-(2-methoxyäthyl)-[1,1'-bicyclohexyl],
trans-4-(3,4-Dichlorphenyl)-trans-4'-(2-äthoxyäthyl)-[1,1'-bicyclohexyl],
trans-4-(4-Chlor-3-fluorphenyl)-trans-4'-(2-äthoxyäthyl)-[1,1'-bicyclohexyl],
4-Chlor-4'-[trans-4-(2-methoxyäthyl)cyclohexyl]biphenyl,
4-Chlor-4'-[trans-4-(2-äthoxyäthyl)cyclohexyl]biphenyl,
3,4-Dichlor-4'-[trans-4-(2-methoxyäthyl)cyclohexyl]-biphenyl,
4-Chlor-3-fluor-4-[trans-4-(2-methoxyäthyl)cyclohexyl]-biphenyl,
4-Chlor-3-fluor-4-[trans-4-(2-äthoxyäthyl)cyclohexyl]-biphenyl,
2-(4-Chlorphenyl)-5-[trans-4-(2-methoxyäthyl)cyclohexyl]-pyridin,
2-(4-Chlorphenyl)-5-[trans-4-(2-äthoxyäthyl)cyclohexyl]-pyridin,
2-(4-Chlorphenyl)-5-[trans-4-(2-methoxyäthyl)cyclohexyl]-pyrimidin,
2-(4-Chlorphenyl)-5-[trans-4-(2-äthoxyäthyl)cyclohexyl]-pyrimidin,
2-(4-Chlor-3-fluorphenyl)-5-[trans-4-(2-methoxyäthyl)-cyclohexyl]pyridin,
2-(4-Chlor-3-fluorphenyl)-5-[trans-4-(2-methoxyäthyl)-cyclohexyl]pyrimidin,
trans-2-(4-Chlorphenyl)-5-[trans-4-(2-methoxyäthyl)-cyclohexyl]-1,3-dioxan.

### Beispiel 4

Die Eigenschaften der Verbindungen der Formel I wurden in binären Mischungen dieser Verbindungen mit 4-(trans-4-Pentylcyclohexyl)benzonitril untersucht. Die Schwellenspannung und die Ansprechzeiten wurden in einer TN-Zelle (low bias tilt) mit 8 mm Plattenabstand bei 22°C gemessen; als Betriebsspannung wurde der 2,5-fache Wert der Schwellenspannung gewählt. Die entsprechenden Daten für reines 4-(trans-4-Pentylcyclohexyl)benzonitril betragen: Klp. (N-I) 54,6°C, V₁₀ = 1,62V, tₒₙ = 30 ms, t_{off} = 42 ms, Δn = 0,120.

### BM-1

90 Gew.-% 4-(trans-4-Pentylcyclohexyl)benzonitril
10 Gew.-% trans-4-(4-Chlorphenyl)-trans-4'-(2-methoxy-äthyl)[1,1'-bicyclohexyl].
Klp. (N-I) 60,1°C, V₁₀ = 1,74V, tₒₙ = 24 ms, t_{off} = 41 ms.

### BM-2

80 Gew.-% 4-(trans-4-Pentylcyclohexyl)benzonitril
20 Gew.-% trans-4-(4-Chlorphenyl)-trans-4'-(2-methoxyäthyl)[1,1'-bicyclohexyl]. Klp. (N-I) 66,5°C, V₁₀ = 1,74V, tₒₙ = 25 ms, t_{off} = 43 ms.

### BM-3

90 Gew.-% 4-(trans-4-Pentylcyclohexyl)benzonitril
10 Gew.-% trans-4-(4-Chlorphenyl)-trans-4'-(3-methoxypropyl)[1,1'-bicyclohexyl].
Klp. (N-I) 64,0°C, V₁₀ = 1,78V, tₒₙ = 24 ms, t_{off} = 39 ms.

### BM-4

80 Gew.-% 4-(trans-4-Pentylcyclohexyl)benzonitril
20 Gew.-% trans-4-(4-Chlorphenyl)-trans-4'-(3-methoxypropyl)[1,1'-bicyclohexyl].
Klp. (N-I) 74,7°C, V₁₀ = 1,88V, tₒₙ = 24 ms, t_{off} = 41 ms.

### BM-5

90 Gew.-% 4-(trans-4-Pentylcyclohexyl)benzonitril
10 Gew.-% 1-Chlor-4-[trans-4-(5-methoxypentyl)cyclohexyl]benzol.
Klp. (N-I) 49,1°C, V₁₀ = 1,52V, tₒₙ = 25 ms, t_{off} = 40 ms.

### BM-6

80 Gew.-% 4-(trans-4-Pentylcyclohexyl)benzonitril
20 Gew.-% 1-Chlor-4-[trans-4-(5-methoxypentyl)cyclohexyl]benzol.
Klp. (N-I) 44,1°C, V₁₀ = 1,53V, tₒₙ = 23 ms, t_{off} = 39 ms.

## Patentansprüche

1. Verbindungen der allgemeinen Formel 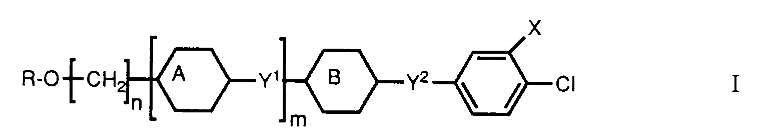 worin R einen Alkylrest mit 1 bis 12 Kohlenstoffatomen bezeichnet; n eine ganze Zahl Von 2 bis 7 ist; Ring A trans-1,4-Cyclohexylen oder 1,4-Phenylen bezeichnet; m entweder 0 oder 1 ist; Ring B trans-1,4-Cyclohexylen, 1,4-Phenylen in welchem gegebenenfalls eine oder zwei nicht benachbarte CH-Gruppen durch Stickstoff ersetzt sind, oder, wenn m für 1 steht auch trans-1,3-Dioxan-2,5-diyl darstellt; Y¹ und Y² unabhängig voneinander eine einfache Kovalenzbindung oder -CH₂CH₂- darstellen; und X Wasserstoff, Fluor oder Chlor bezeichnet.

2. Verbindungen nach Anspruch 1, dadurch gekennzeichnet, dass R einen geradkettigen Alkylrest mit 1 bis 5, vorzugsweise 1 bis 3 Kohlenstoffatomen darstellt.

3. Verbindungen nach Anspruch 1 oder 2, dadurch gekennzeichnet, dass Ring A trans-1,4-Cyclohexylen darstellt.

4. Verbindungen nach Anspruch 1, 2 oder 3, dadurch gekennzeichnet, dass Ring B trans-1,4-Cyclohexylen oder 1,4-Phenylen bezeichnet.

5. Verbindungen nach einem der Ansprüche 1 bis 4, dadurch gekennzeichnet, dass mindestens eine der Gruppen Y¹ und Y² eine einfache Kovalenzbindung darstellt.

6. Flüssigkristalline Mischungen von mindestens 2 Komponenten, dadurch gekennzeichnet, dass mindestens eine der Komponenten eine Verbindung der allgemeinen Formel 1 ist.

7. Verwendung der Verbindungen der allgemeinen Formel I für elektro-optische Zwecke.

## Claims

1. Compounds of the general formula 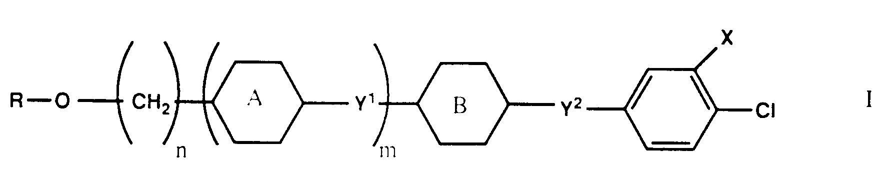 wherein R denotes an alkyl residue with 1 to 12 carbon atoms; n is a whole number of 2 to 7, ring A denotes trans-1,4-cyclohexylene or 1,4-phenylene; m is either 0 or 1; ring B is trans-1,4-cyclohexylene, 1,4-phenylene in which optionally one CH group or two non-adjacent CH groups is/are replaced by nitrogen or when m stands for 1, also trans-1,3-dioxane-2,5-diyl; Y¹ and Y² each independently represent a single covalent bond or -CH₂CH₂-; and X denotes hydrogen, fluorine or chlorine.

2. Compounds according to claim 1, characterized in that R repesents a straight-chain alkyl residue with 1 to 5, preferably 1 to 3, carbon atoms.

3. Compounds according to claim 1 or 2, characterized in that ring A represents trans-1,4-cyclohexylene.

4. Compounds according to claim 1, 2 or 3, characterized in that ring B denotes trans-1,4-cyclohexylene or 1,4-phenylene.

5. Compounds according to any one of claims 1 to 4, characterized in that at least one of the groups Y¹ and Y² represents a single covalent bond.

6. Liquid crystalline mixtures of at least 2 components, characterized in that at least one component is a compound of general formula 1.

7. The use of the compounds of general formula I for electro-optical purposes.

## Revendications

1. Composés de formule générale 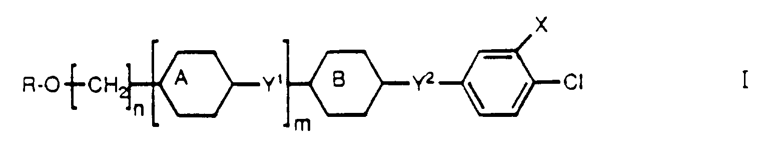 dans laquelle R représente un groupe alkyle en C1-C12 ; n est un nombre entier allant de 2 à 7 ; le cycle A est un cycle trans-1,4-cyclohexylène ou 1,4-phénylène ; m est égal à 0 ou 1 ; le cycle B est un cycle trans-1,4,cyclohexylène, 1,4-phénylène dans lequel, le cas échéant, un ou deux groupes CH non voisins sont remplacés par l'azote ou bien encore, lorsque m = 1, un cycle trans-1,3-dioxanne-2,5-diyle ; Y¹ et Y² représentent chacun, indépendamment l'un de l'autre, une liaison covalente simple ou un groupe -CH₂CH₂- ; et X représente l'hydrogène, le fluor ou le chlore.

2. Composés selon la revendication 1, caractérisés en ce que R représente un groupe alkyle à chaîne droite en C1-C5, de préférence en C1-C3.

3. Composés selon la revendication 1 ou 2, caractérisés en ce que le cycle A est un cycle trans-1,4-cyclohexylène.

4. Composés selon la revendication 1, 2 ou 3, caractérisés en ce que le cycle B est un cycle trans-1,4-cyclohexylène ou 1,4-phénylène.

5. Composés selon l'une des revendications 1 à 4, caractérisés en ce que l'un au moins des symboles Y¹ et Y² représente une liaison covalente simple.

6. Mélanges à cristaux liquides à au moins deux composants caractérisés en ce que l'un au moins des composants est un composé de formule générale I.

7. Utilisation des composés de formule générale I dans des applications électro-optiques.
